# EUROPEAN PATENT SPECIFICATION

(11) **EP 0 878 180 B1**
(45) Date of publication and mention of the grant of the patent: **09.04.2003**
(21) Application number: 97116254.0
(22) Date of filing: 18.09.1997
(51) Int. Cl.: A61F 13/15

(54) **Disposable pull-on type diaper with seal lines in front**
Wegwerfwindel zum Überziehen mit Säumen auf der Vorderseite
Couche-culotte jetable avec coutures antérieures

(30) Priority: 13.05.1997 JP 13773097
(43) Date of publication of application: 18.11.1998
(73) Proprietor: Crecia Corporation, Shinjuku-ku, Tokyo 163-11 (JP)
(72) Inventor: Saito, Katsuhiko, c/o Crecia Corporation, Shinjuku-ku, Tokyo 163-11 (JP); Matsubara, Hiroaki, c/o Crecia Corporation, Shinjuku-ku, Tokyo 163-11 (JP)
(74) Representative: VOSSIUS & PARTNER

(56) References cited:
- EP-A- 0 023 804
- EP-A- 0 522 584
- US-A- 3 332 432
- US-A- 4 205 679

## Description

The present invention relates to a disposable pull-on type diaper, in particular, one having seal line zones in front, that is, on the abdominal side.

EP 0 522 584 A2 discloses a diaper having an elastic section preventing a front region from moving downwards while being worn.

EP 0 023 804 A1 discloses a diaper comprising an elastic member disposed between first and second substrates of flexible gatherable material, said elastic member including a plurality of interconnected elastic elements defining apertures therebetween.

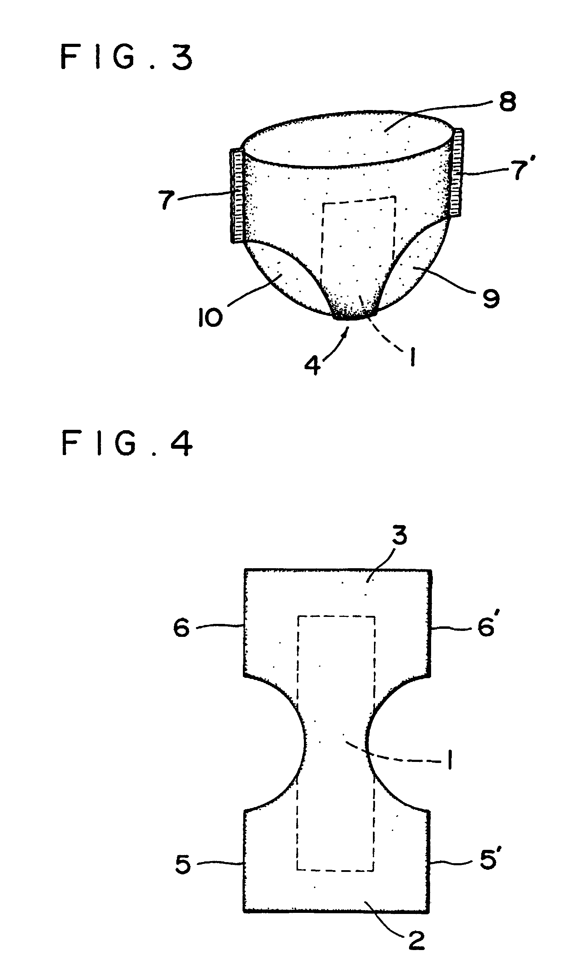

The disposable pull-on type diaper has a constitution, in which the outer surface is covered with a liquid-impermeable sheet and the inner surface is covered with a liquid-permeable sheet, with an absorbent interposed therebetween. And, in the shape, as shown in Fig. 3, it has a trunk portion aperture (8) along the upper end line and thigh portion apertures (9), (10) for both legs along the right and left lower end lines in similar to the shape of usual underwear briefs. Further, it has the absorbent (1) located in the crotch side sheet region (4) and seal line zones (7), (7') along the both end lines of waist portion which are formed inevitably in course of the manufacturing said diaper.

These seal line zones are linear ones being in an adhered overlapping state, resulting at the time of mutually adhering the one side edge zone (5) of the abdominal side sheet region (2) which becomes front side on wearing and the same one side edge zone (6) of the back side sheet region (3) which becomes back side on wearing, and the another side edge zone (5') of the abdominal side sheet region (2) and the same another side edge zone (6') of the back side sheet region (3) respectively by heat sealing etc., in case developed diaper materials of which central region of the longitudinal direction being pinched in and the respective front and behind shapes of longitudinal direction being laterally in approximate symmetry is converted to the disposable pull-on type diaper, as shown in Fig. 4, wherein the liquid-permeable sheet and the liquid-impermeable sheet being constituting materials are in a state of being hardened with heat, adhesive, etc.

Such disposable pull-on type diaper is worn not only by babies, but also by adults who become bed-ridden due to an accident, disease, etc. and aged persons whose health is declining, and the like. However, since particularly adults, aged persons, etc. confined to bed for a long time are in a state of being unable to toss about in bed by their own power, the direction of their body is being occasionally changed by their carers. Namely, they are repeating to turn over on their back and to turn right and left sideways, but, when they are left to be sideways for a long time, in particular, then the side seal of said diaper is pressed against their skin, causing discomfort for wearers of said disposable pull-on type diaper, and further there has been an anxiety to result in a cause of generating bed sores.

Moreover, when taking off the disposable pull-on type diaper from wearers, there are a case of bringing it down toward their toes as it is and another case that their carers rupture it along seal lines by hands to develop in plane shape and remove. And, the excreta of wearers in the state of being bed-ridden sometimes reaches the seal lines, hence the rupture along seal lines by carers could not be said to be sanitary.

The invention improves such defects of conventional disposable pull-on type diaper and provides a constitution not to give uncomfortable feeling to wearers.

The disposable pull-on type diaper of the invention is one as defined in independent claim 1. Claims 2-4 disclosed preferred embodiments of the inventive diaper. Claim 5 discloses a manufacturing process of the diaper according to claims 1-4.

### BRIEF DESCRIPTION OF THE DRAWING

Fig. 1 is a developed plan showing an example of the invention.

Fig. 2 is an assembling oblique view of the same.

Fig. 3 is an assembling oblique view of a conventional example.

Fig. 4 is a developed plan of the same.

Fig. 5 is an outside view of the example of the invention.

Fig. 6 is A arrow direction view of Fig. 5, showing a condition of "on wearing".

Fig. 7 is B arrow direction view of Fig. 5, showing a condition of "on wearing".

Fig. 8 is one corresponding to Fig. 7, showing the conventional example.

### DETAILED DESCRIPTION OF THE INVENTION

In this way, by providing the seal line zones inside the right and left iliac crests of human body, that is, inside the bone crests which stick out at the right and left of the abdominal side in the waist position of human body, in case a person, even a bed-ridden person, may have the direction of his body placed sideways for a long time, the hard seal line zones are not pressed against his body, thus allowing him to spend comfortably.

And, when the seal line zones come on the abdominal side as in the invention, then the area of back side sheet region becomes wider, resulting in a structure to largely wrap the hip portion of wearers therein, and the excreta reaching the seal line zones become less even in the state of being bed-ridden, which is sanitary for the carers. Further, when removing the diaper, after the diaper is ruptured along the seal line zones and developed, the excreta is wrapped up easily and can be disposed promptly because of wider back side sheet region, thus making it possible to prevent smell of the excreta diffusing around and dispose more sanitarily.

Moreover, when rupturing the diaper on wearing along the seal line zones, said seal line zones appear usually on the upper side in case of the inventive diaper, hence it is easy to handle it for the wearer and carers, leading to good handling.

Furthermore, according to the inventive diaper, distinction of the front and behind sides is possible at a glance from external appearance, thus mistake in front or behind side on wearing becoming less.

Besides, for the disposable pull-on type diaper for adults and aged persons, these seal line zones are provided within 10 cm, more preferably within 8 cm on the both sides from the longitudinal center line of the abdominal region of said diaper, respectively.

### Example

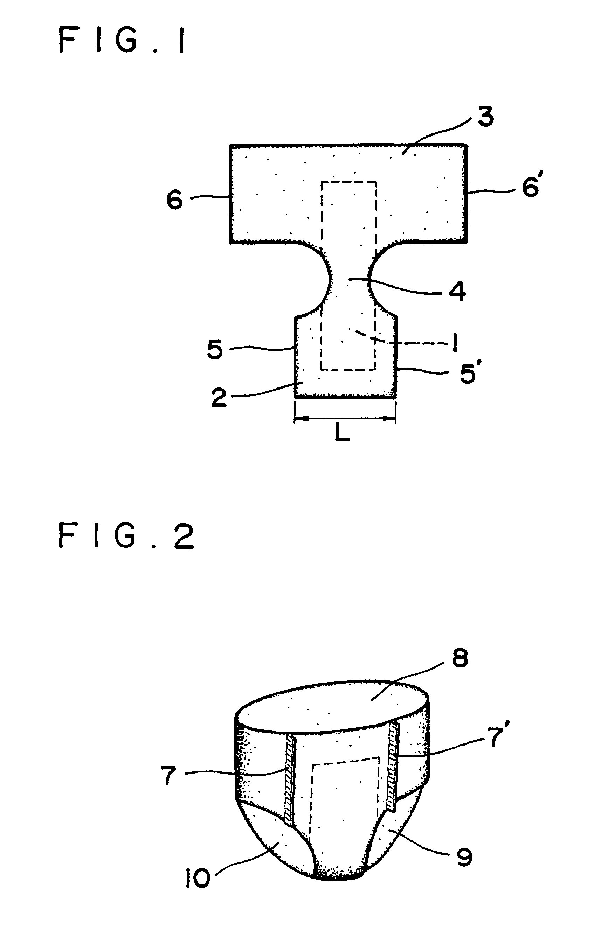

As shown in Fig. 1, an approximately rectangular absorbent (1) was interposed between a liquid-impermeable sheet of the outer surface and a liquid-permeable sheet of the inner surface to be fixed therewith, and a abdominal side sheet region (2) being one end portion in the longitudinal direction and a back side sheet region (3) being another end portion in the longitudinal directions, wherein said back side sheet region (3) is laterally longer than said abdominal side sheet region (2), were continued through a crotch side sheet region (4) of which both lateral sides are curved in oppositely towards the insides, thereby making a developed diaper material in a shape of almost a sandglass. The longitudinal length of zone (5), (5') is the same as that of zone (6), (6'). At this time, the lateral dimension (L) of the abdominal side sheet region (2) was made to be about 16 cm.

And, said diaper material was bent in the crotch side sheet region (4) so that the surface of liquid-permeable sheet became inside, and the both side edge zones (5), (5') of the abdominal side sheet region (2) and the respective opposed side edge zones (6), (6') of the back side sheet region (3) were adhered by means of heat fusion to provide the seal line zones (7), (7') on the abdominal side, (that is, zones (5) and (6) were overlapped and adhered each other to afford seal line zone (7) and zones (5') and (6') were likewise overlapped and adhere each other to afford seal line zone (7')) as shown in Fig. 2, thereby making up a disposable pull-on type diaper with the trunk portion aperture (8) formed along the upper end line and the thigh portion apertures (9), (10) formed along the right and left lower end lines.

Since such a disposable pull-on type diaper has a distance (L) between seal line zones (7), (7') being about 16 cm, the seal line zones come inside the hard iliac crests located at the right end and left end of the abdominal side of adult on wearing, thus do not giving any uncomfortable feeling to its wearers.

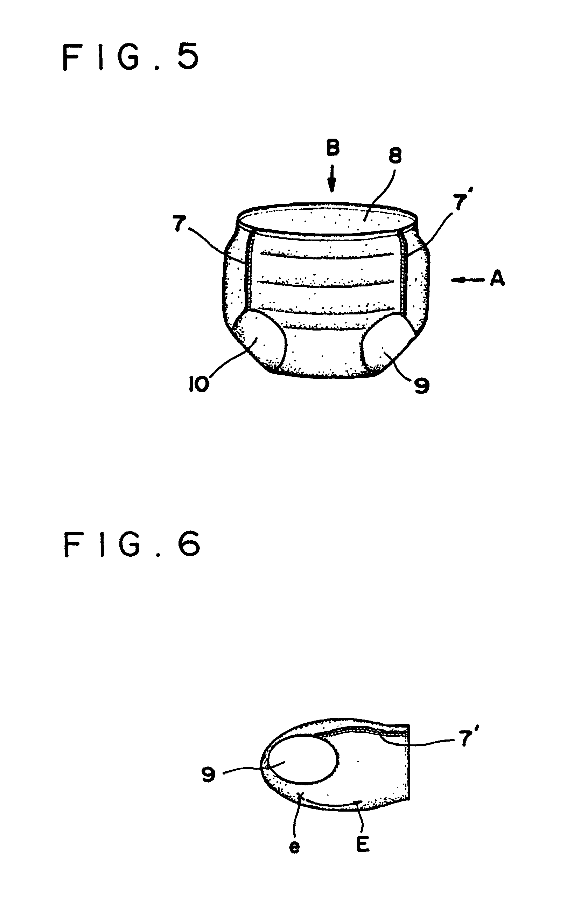

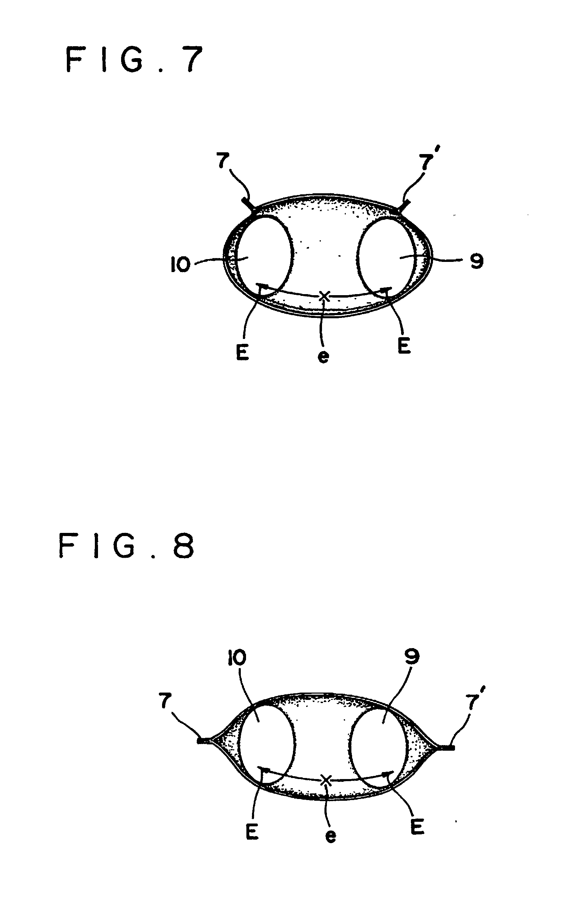

As illustrated in Fig. 6 and Fig. 7 showing the diaper of the present invention, excreta (e) excreted by the wearers lying down on bed inside the worn diapers are in tendency to move along the downside, i.e. in the direction of arrow (E), but the excreta (e) would neither reach nor contact the seal line zones (7), (7') because the seal lines are located up sides of the diaper and enough apart from the line of arrow (E).

On the contrary, in Fig. 8 showing the conventional diaper, the seal line zones (7), (7') are located on the extension lines of arrows (E) (E) and hence the excreta (e) would readily reach and contact the seal line zones (7), (7').

As described above, in accordance with the invention, since the seal lines to be formed inevitably in course of making disposable pull-on type diaper are present on the abdominal side, these hard seal lines are never pressed against the skin for wearer who cannot toss about by himself, even if his body might be placed in any direction, thus decreasing the causes of bedsore, decubitus, etc. and allowing him to spend comfortably. Moreover, also when taking off the diaper by rupturing by hands along the seal lines, the excreta reaching said seal lines become less, because of wider back side sheet region, and yet it is possible to wrap the excreta therein promptly, thus leading to sanitary disposal. Furthermore, wearing mistake in front or behind becomes less at the time of wearing the diaper and, also when rupturing diaper, the seal line zones always come on the upper surface and hence the handling the diaper becomes easy. The invention exerts such effects and others.

## Claims

1. A disposable pull-on type diaper having seal line zones (7,7') in front wherein the abdominal side sheet region (2) and the back side sheet region (3) continue through the crotch side sheet region (4), and respective opposed side edge zones (5,5',6,6') of the both abdominal side sheet region (2) and back side sheet region (3) are overlapped and adhered each other to form a trunk portion aperture (8) along the upper end line and thigh portion apertures (9,10) along the right and left lower end lines, respectively, of said disposable pull-on type diaper, **characterized in that** the abdominal side sheet region (2) and the back side sheet region (3) are formed respectively in the same constitution with the same materials, and the right and left seal line zones (7,7') to be formed by overlapping and adhering those respective opposed abdominal and back side edge zones (5,5',6,6') each other, so that the body facing surfaces are adhered together to form the seal lines (7,7') projecting outwardly are provided inside the position corresponding to the right and left iliac crests of the wearer of said diaper on the abdominal side thereof, and the above right and left seal line zones (7,7') define straight lines extending in the longitudinal direction further the seal line zones (7,7') are provided within 10 cm from the longitudinal center line of the abdominal side of diaper toward the both sides thereof, respectively.

2. The disposable pull-on type diaper of Claim 1 , wherein the outer surface of the diaper is covered with a liquid-impermeable sheet and the inner surface of the diaper is covered with a liquid-permeable sheet, with an absorbent (1) interposed therebetween.

3. The disposable pull-on type diaper of anyo ne of Claims 1 and 2, wherein the respective opposed side edge zones (5,5',6,6') of the liquid-permeable sheet and the liquid-impermeable sheet consisting materials of the diaper are overlapped and adhered with the applied adhesive each other under application of heating and pressing.

4. The disposable pull-on type diaper of anyone of Claims 1-3, wherein the diaper is made of a sheet material having a developed shape consisting of a broad rectangular region, a narrow rectangular region and an intermediate pinched-in region to form a nearly sandglass-shape as a whole, and both the opposed side edge zones (5,5') of the abdominal side sheet region (2) of the narrow rectangular region and the back side sheet region (6,6') of the broad rectangular region are overlapped and adhered each other respectively to afford a disposable pull-on type diaper having seal line zones (7,7') located on the abdominal side thereof.

5. A manufacturing process of the disposable pull-on type diaper of any one of claims 1-4 comprising a step of preparing a sheet material for diaper having a developed shape consisting of a broad rectangular region, a narrow rectangular region and an intermediate pinched-in region to form a nearly sandglass-shape as a whole, wherein the sheet material consists of a liquid-impermeable sheet covering the outer surface of the diaper, a liquid-permeable sheet covering the inner surface of the diaper and an absorbent (1) interposed therebetween, and a step of overlapping and adhering both the opposed side edge zones (5,5',6,6') of the abdominal side sheet region (2) and the back side sheet region (3) to afford a disposable pull-on type diaper having seal line zones (7,7') located on the abdominal side thereof, **characterized in that** the abdominal side sheet region (2) and the back side sheet region (3) are formed respectively in the same constitution with the same materials, and the seal line zones to be formed by overlapping and adhering those respective opposed side edge zones (5,5',6,6') each other are provided inside the position corresponding to the right and left iliac crests of the wearer of said diaper on the abdominal side thereof.

## Patentansprüche

1. Wegwerfwindel zum Überziehen mit Saumzonen (7, 7') auf der Vorderseite, wobei der bauchseitige Schichtbereich (2) und der rückenseitige Schichtbereich (3) sich durch den leistenbeugenseitigen Schichtbereich (4) fortsetzen und jeweilige entgegengesetztseitge Randzonen (5, 5', 6, 6') sowohl des bauchseitigen Schichtbereichs (2) als auch des rückenseitigen Schichtbereichs (3) überlappt und aneinander geklebt werden, um eine Öffnung (8) für den Rumpfteil entlang der oberen Endlinie und Öffnungen (9, 10) für die Oberschenkel jeweils entlang den rechten und linken unteren Endlinien der genannten Wegwerfwindel zum Überziehen zu bilden, **dadurch gekennzeichnet, daß** der bauchseitige Schichtbereich (2) und der rückenseitige Schichtbereich (3) jeweils mit dem gleichen Aufbau aus den gleichen Materialien hergestellt sind und daß die rechten und linken Saumzonen (7, 7'), die gebildet werden sollen, indem diese jeweiligen entgegengesetzten seitlichen bauch- und rückenseitigen Randzonen (5, 5', 6, 6') überlappt und aneinander geklebt werden, so daß die dem Körper zugewandten Oberflächen aneinander geklebt werden, um nach außen vorstehende Säume (7, 7') zu bilden, innerhalb der Position vorgesehen werden, die den linken und rechten Darmbeinkämmen des Trägers der Windel auf deren Bauchseite entspricht, und daß die obigen rechten und linken Saumzonen (7, 7') gerade Linien definieren, die sich in der Längsrichtung erstrecken, ferner sind die Saumzonen (7, 7') jeweils innerhalb von 10 cm von der Längsmittellinie der Bauchseite der Windel in Richtung ihrer beiden Seiten vorgesehen.

2. Wegwerfwindel zum Überziehen nach Anspruch 1, wobei die äußere Oberfläche der Windel mit einer flüssigkeitsundurchlässigen Schicht bedeckt ist und die innere Oberfläche der Windel mit einer flüssigkeitsdurchlässigen Schicht bedeckt ist, wobei ein Absorptionsmaterial (1) dazwischen angeordnet ist.

3. Wegwerfwindel zum Überziehen nach einem der Ansprüche 1 oder 2, wobei die jeweiligen entgegengesetztseitigen Randzonen (5, 5', 6, 6') der flüssigkeitsdurchlässigen Schicht und der flüssigkeitsundurchlässigen Schicht, aus deren Materialien die Windel besteht, überlappt und mit dem aufgetragenen Klebstoff unter Anwendung von Erwärmung und Drücken aneinander geklebt werden.

4. Wegwerfwindel zum Überziehen nach einem der Ansprüche 1 bis 3, wobei die Windel aus einem Schichtmaterial mit einer entwickelten Form gefertigt ist, welche aus einem breiten rechteckigen Bereich, einem schmalen rechteckigen Bereich und einem verschmälerten Zwischenbereich besteht, um als Ganzes nahezu eine Sanduhrform zu bilden, und wobei beide entgegengesetztseitigen Randzonen (5, 5') des bauchseitigen Schichtbereichs (2) des schmalen rechteckigen Bereichs und des rückenseitigen Schichtbereichs (6, 6') des breiten rechteckigen Bereichs jeweils überlappt und aneinander geklebt werden, um eine Wegwerfwindel zum Überziehen mit auf ihrer Bauchseite angeordneten Saumzonen (7, 7') zu bieten.

5. Herstellungsverfahren für die Wegwerfwindel zum Überziehen nach einem der Ansprüche 1 bis 4, das aufweist: einen Schritt zum Herstellen eines Schichtmaterials für Windeln mit einer entwickelten Form, welches aus einem breiten rechteckigen Bereich, einem schmalen rechteckigen Bereich und einem verschmälerten Zwischenbereich besteht, um als Ganzes nahezu eine Sanduhrform zu bilden, wobei das Schichtmaterial aus einer die äußere Oberfläche der Windel bedeckenden flüssigkeitsundurchlässigen Schicht und einer die innere Oberfläche der Windel bedeckenden flüssigkeitsdurchlässigen Schicht und einem dazwischen angeordneten Absorptionsmaterial (1) besteht, und einen Schritt zum Überlappen und Kleben der beiden entgegengesetztseitigen Randzonen (5, 5', 6, 6') des bauchseitigen Schichtbereichs (2) und des rückenseitigen Schichtbereichs (3), um eine Wegwerfwindel zum Überziehen mit auf ihrer Bauchseite angeordneten Saumzonen (7, 7') zu bieten, **dadurch gekennzeichnet, daß** der bauchseitige Schichtbereich (2) und der rückenseitige Schichtbereich (3) jeweils mit dem gleichen Aufbau aus den gleichen Materialien hergestellt sind und daß die Saumzonen (7, 7'), die gebildet werden sollen, indem diese jeweiligen entgegengesetztseitigen Randschichtzonen (5, 5', 6, 6') überlappt und aneinander geklebt werden, innerhalb der Position vorgesehen werden, die den linken und rechten Darmbeinkämmen des Trägers der Windel auf deren Bauchseite entspricht.

## Revendications

1. Couche de type jetable en tirant dessus ayant des zones de lignes d'étanchéité (7, 7') à l'avant, dans laquelle la région de feuille latérale abdominale (2) et la région de feuille latérale arrière (3) sont continues à travers la région de feuille latérale de béquille (4), et des zones de bords latéraux opposées respectives (5, 5', 6, 6') à la fois de la région de feuille latérale abdominale (2) et de la région de feuille latérale arrière (3) se chevauchent et collent les unes aux autres pour former une ouverture de partie de tronc (8) le long de la ligne d'extrémité supérieure et des ouvertures de parties de cuisses (9, 10) le long des lignes d'extrémités inférieures gauche et droite, respectivement, de ladite couche de type jetable en tirant dessus, **caractérisée en ce que** la région de feuille latérale abdominale (2) et la région de feuille latérale arrière (3) sont respectivement formées suivant la même constitution avec les mêmes matériaux, et les zones de lignes d'étanchéité droite et gauche (7, 7'), à former par chevauchement et collage de ces zones de bords latéraux opposées abdominale et arrière (5, 5', 6, 6') les unes avec les autres pour que les surfaces opposées au corps collent ensemble pour former les lignes d'étanchéité (7, 7') faisant saillie vers l'extérieur, sont fournies à l'intérieur de la position correspondant aux crêtes iliaques droite et gauche du porteur de ladite couche sur le côté abdominal de celle-ci, et les zones de lignes d'étanchéité droite et gauche (7, 7') ci-dessus définissent des lignes droites s'étendant dans la direction longitudinale, les zones de lignes d'étanchéité (7, 7') sont en outre fournies dans les 10 cm à partir de la ligne centrale longitudinale du côté abdominal de la couche vers les deux côtés de celle-ci, respectivement.

2. Couche de type jetable en tirant dessus selon la revendication 1, dans laquelle la surface externe de la couche est recouverte d'une feuille imperméable au liquide et la surface interne de la couche est recouverte d'une feuille perméable au liquide avec un absorbant (1) intercalé entre celles-ci.

3. Couche de type jetable en tirant dessus selon l'une quelconque des revendications 1 et 2, dans laquelle les zones de bords latéraux opposées respectives (5, 5', 6, 6') de la feuille perméable au liquide et des matériaux constituant la feuille imperméable au liquide de la couche se chevauchent et collent avec l'adhésif appliqué les unes aux autres sous l'application de chaleur et de pression.

4. Couche de type jetable en tirant dessus selon l'une quelconque des revendications 1-3, dans laquelle la couche est constituée d'un matériau de feuille ayant une forme développée constituée d'une large région rectangulaire, d'une région rectangulaire étroite et d'une région pincée intermédiaire pour former une forme proche d'un sablier dans sa globalité, et à la fois les zones de bords latéraux opposées (5, 5') de la région de feuille latérale abdominale (2) de la région rectangulaire étroite et de la région de feuille latérale arrière (6, 6') de la large région rectangulaire se chevauchent et collent les unes aux autres respectivement pour fournir une couche de type jetable en tirant dessus ayant des zones de lignes d'étanchéité (7,7') disposées sur le côté abdominal de celle-ci.

5. Procédé de fabrication de la couche de type jetable en tirant dessus selon l'une quelconque des revendications 1-4 comprenant une étape de préparation d'un matériau en feuille pour couche ayant une forme développée constituée d'une large région rectangulaire, d'une région rectangulaire étroite et d'une région pincée intermédiaire pour former une forme proche du sablier dans sa globalité, dans lequel le matériau de feuille est constitué d'une feuille imperméable au liquide recouvrant la surface externe de la couche, d'une feuille perméable au liquide recouvrant la surface interne de la couche et d'un absorbant (1) intercalé entre celles-ci, et une étape de chevauchement et de collage des deux zones de bords latéraux opposées (5, 5', 6, 6') de la région de feuille latérale abdominale (2) et de la région de feuille latérale arrière (3) pour fournir une couche de type jetable en tirant dessus ayant des zones de lignes d'étanchéité (7, 7') disposées sur le côté abdominal de celle-ci, **caractérisé en ce que** la région de feuille latérale abdominale (2) et la région de feuille latérale arrière (3) sont respectivement formées suivant la même constitution avec les mêmes matériaux, et les zones de lignes d'étanchéité à former par chevauchement et collage de ces zones de bords latéraux opposées respectives (5, 5', 6, 6') les unes avec les autres sont fournies à l'intérieur de la position correspondant aux crêtes iliaques droite et gauche du porteur de ladite couche sur le côté abdominal de celle-ci.
